Europäisches Patentamt

European Patent Office

Office européen des brevets

(11) Veröffentlichungsnummer: **0 357 146**
**A2**

## (12) EUROPÄISCHE PATENTANMELDUNG

(21) Anmeldenummer: 89202193.2

(22) Anmeldetag: 30.08.89

(51) Int. Cl.5: **G21K 1/04 , A61B 6/02**

(30) Priorität: 01.09.88 DE 3829688

(43) Veröffentlichungstag der Anmeldung:
07.03.90 Patentblatt 90/10

(84) Benannte Vertragsstaaten:
DE FR GB IT SE

(71) Anmelder: **Philips Patentverwaltung GmbH**
**Wendenstrasse 35 Postfach 10 51 49**
**D-2000 Hamburg 1(DE)**

(84) .DE

Anmelder: **N.V. Philips' Gloeilampenfabrieken**
**Groenewoudseweg 1**
**NL-5621 BA Eindhoven(NL)**

(84) FR GB IT SE

(72) Erfinder: **Harding, Geoffrey, Dr.**
**Franzosenkoppel 110**
**D-2000 Hamburg-Norderstedt(DE)**
Erfinder: **Kosanetzky, Joesef-Maria, Dr.**
**Waldstrasse 78 A**
**D-2000 Norderstedt(DE)**
Erfinder: **Fischer, Karl-Heinz**
**Kriegerdankweg 18**
**D-2000 Hamburg 61(DE)**
Erfinder: **Meyer, Alfred Günter**
**Novalisweg 9**
**D-2000 Hamburg 60(DE)**

(74) Vertreter: **Hartmann, Heinrich, Dipl.-Ing.**
**Philips Patentverwaltung GmbH**
**Wendenstrasse 35 Postfach 10 51 49**
**D-2000 Hamburg 1(DE)**

(54) Anordnung zur Erzeugung eines Röntgen- oder Gammastrahls mit geringem Querschnitt und veränderlicher Richtung.

(57) Die Erfindung betrifft eine Anordnung zur Erzeugung eines Röntgen- oder Gammastrahls (11) mit geringem Querschnitt und veränderlicher Richtung, mit einer ein Strahlenbündel liefernden Röntgen- oder Gammastrahlenquelle (1) und einer Blendenanordnung, die aus dem Strahlenbündel den Röntgenstrahl ausblendet, und die ein feststehendes Blendenteil (7) mit einem geradlinigen Schlitz (8) und einen um eine Drehachse (5) rotierenden zylinderförmigen ersten Blendenkörper (3) mit einem auf seinem Mantel spiralförmig umlaufenden Schlitz (9) umfaßt. Zur Verringerung des Herstellungsaufwandes und zur Schaffung einer auch für unterschiedliche Abstände zwischen Strahlenquelle und Drehachse geeigneten Anordnung ist dabei vorgesehen, daß der Blendenkörper (3) zumindest über einen Teil seiner Länge einen wenigstens näherungsweise halbkreisförmigen Querschnitt hat.

Fig.1

## Anordnung zur Erzeugung eines Röntgen- oder Gammastrahls mit geringem Querschnitt und veränderlicher Richtung

Die Erfindung betrifft eine Anordnung zur Erzeugung eines Röntgen- oder Gammastrahls mit geringem Querschnitt und veränderlicher Richtung, mit einer ein Strahlenbündel liefernden Röntgen- oder Gammastrahlenquelle und einer Biendenanordnung, die aus dem Strahlenbündel den Röntgenstrahl ausblendet, und die ein feststehendes Blendenteil mit einem geradlinigen Schlitz und einen um eine Drehachse rotierenden zylinderförmigen ersten Blendenkörper mit einem auf seinem Mantel spiralförmig umlaufenden Schlitz umfaßt

Anordnungen dieser Art sind aus der EP-OS 74 021 für medizinische Anwendungen und aus der DE-OS 34 43 095 für industrielle Anwendungen im wesentlichen bekannt. Der Blendenkörper wird dabei durch einen Hohlzylinder aus einem die Strahlung absorbierenden Material gebildet, der auf seinem Umfang mit zwei gegeneinander versetzten spiralförmig umlaufenden Schlitzen versehen ist. Wenn auf einen solchen Blendenkörper senkrecht zu dessen Zylinderachse ein Bündel paralleler Strahlen fällt, dann gibt es stets einen Punkt, in dem ein Röntgenstrahl die beiden Schlitze passiert. Wird der Blendenkörper gedreht, dann wandert dieser Punkt auf der Achse entlang, so daß hinter dem Blendenkörper ein periodisch bewegter Röntgenstrahl austritt. Dieser Periodisch bewegte Röntgenstrahl kann für medizinische oder industrielle Untersuchungen benutzt werden. Der feststehende Blendenteil hat dabei die Aufgabe, die Röntgenstrahlung senkrecht zu der Verschiebungsrichtung des Röntgenstrahls definiert zu begrenzen.

In der Praxis werden Röntgenstrahlen mit einer Röntgenröhre erzeugt, die ein Bündel divergenter Strahlen liefert. Bei einem divergierenden Strahlenbündel ergibt sich aber zu den Rändern hin eine Abnahme der Intensität des Röntgenstrahls. Diese kann zumindest teilweise dadurch verhindert werden, daß die Schlitze in geeigneter Weise geformt werden und daß die Drehachse des Blendenkörpers nicht exakt parallel zu der durch den geradlinigen Schlitz und die Strahlenquelle gebildeten Ebene verläuft, sondern mit dieser einen Winkel einschließt, der von der Divergenz des Strahlenbündels, d.h. von dem Abstand der Strahlenquelle von der Drehachse des Blendenkörpers, abhängt.

Ein solcher Blendenkörper ist daher nur mit erheblichem Aufwand herzustellen und nur für einen bestimmten Abstand zwischen Strahlenquelle und Drehachse geeignet.

Aufgabe der vorliegenden Erfindung ist es, eine Anordnung der eingangs genannten Art zu schaffen, die einen hin- und hergehenden Primärstrahl auf andere Weise erzeugt.

Diese Aufgabe wird erfindungsgemäß dadurch gelöst, daß der Blendenkörper zumindest über einen Teil seiner Länge einen wenigstens näherungsweise halbkreisförmigen Querschnitt hat.

Bei der Erfindung wird also der Röntgenstrahl durch das Zusammenwirken des spiralförmigen Schlitzes im ersten Blendenkörper und des geradlinigen Schlitzes im feststehenden Blendenteil aus dem (divergenten) Röntgenstrahlenbündel erzeugt. Durch die Rotation des ersten Blendenkörpers verlagert sich der Austrittspunkt des Röntgenstrahls, so daß ein Periodisch bewegter Röntgenstrahl resultiert. Weil die Röntgenstrahlung durch einen Schlitz austritt und nicht durch zwei gegenüber liegende Schlitze im Blendenkörper, fällt die Intensität des Röntgenstrahls zu den Enden des Blendenkörpers hin nicht ab. Es ist also nicht erforderlich, dem Schlitz eine bestimmte Form zu geben oder ihn in definierter Weise gegenüber dem geradlinigen Schlitz anzuordnen.

Durch das Zusammenwirken der Schlitze im feststehenden Blendenteil und im Blendenkörper wird ein Röntgenstrahl mit trapezförmigem Querschnitt definiert. Erwünscht ist jedoch ein quadratischer (bzw. ein kreisförmiger) Querschnitt, wodurch sich ein richtungsunabhängiges räumliches Auflösungsvermögen ergeben würde. Bei gleicher Breite der beiden Schlitze ist die Annäherung an die quadratische Querschnittsform um so besser, je größer der Winkel ist, unter dem die beiden Schlitze einander schneiden. Ein großer Schnittwinkel könnte durch Verwendung eines Blendenkörpers mit großem Durchmesser und kleiner axialer Länge erreicht werden. Für viele Anwendungen ist jedoch ein relativ großer Ablenkwinkel des Röntgenstrahls erforderlich, was eine entsprechende axiale Länge des Blendenkörpers voraussetzt; ein großer Durchmesser ist bei vielen Anwendungen wegen des damit verbundenen Bauvolumens unerwünscht.

Um auch bei einem Blendenkörper mit relativ großer axialer Länge und relativ geringem Durchmesser einen günstigen Strahlquerschnitt zu erreichen, sieht eine Weiterbildung der Erfindung vor, daß in dem ersten Blendenkörper in axialer Richtung mehrere spiralförmig umlaufende Schlitze aufeinander folgen. Bei n Schlitzen im ersten Blendenkörper erstreckt sich jeder Schlitz nur über 1/n der Länge des Blendenkörpers, so daß die Projektion der spiralförmigen Schlitze im Blendenkörper auf den Blendenteil den darin vorgesehenen Schlitz jeweils unter einem relativ großen Winkel schneidet, was eine günstige Querschnittsform ergibt.

Dabei werden durch die n spiralförmigen Schlitze n Röntgenstrahlen erzeugt, die bei einer halben Umdrehung des Blendenkörpers jeweils in eine Position wandern, die zu Beginn ein benachbarter Röntgenstrahl einnahm Bei verschiedenen Anwendungen - beispielsweise bei solchen, bei denen die von dem Röntgenstrahl erzeugte Streustrahlung gemessen wird, wie in der DE-OS 34 43 095 beschrieben - will man jedoch nur mit einem einzigen Röntgenstrahl arbeiten

Eine hierfür geeignete weitere Ausgestaltung der Erfindung sieht vor, daß ein zweiter Blendenkörper mit halbkreisförmigem Querschnitt über wenigstens einen Teil seiner Länge vorgesehen ist, daß die beiden Blendenkörper koaxial und einander umschließend angeordnet sind, daß der erste Blendenkörper entsprechend der Zahl der darin vorgesehenen Schlitze schneller rotiert als der zweite Blendenkörper, und daß die Anordnung und die Form der Öffnung(en) auf dem Umfang des zweiten Blendenkörpers derart ist, daß jeweils nur durch einen der Schlitze (z.B. 9b) ein nutzbarer Strahl austreten kann.

Die einfachste Ausgestaltung dieser Weiterbildung der Erfindung sieht vor, daß in dem zweiten Blendenkörper als einzige Öffnung ein spiralförmiger Schlitz vorgesehen ist, der wesentlich breiter ist als die Schlitze in dem ersten Blendenkörper und der sich über einen Umfangswinkel von zumindest annähernd 180° erstreckt. Hierbei werden die Schlitze im Laufe einer halben Umdrehung des zweiten Blendenkörpers nacheinander durchstrahlt, wobei sich der Röntgenstrahl kontinuierlich von der einen zur anderen Seite bewegt. Da die Umdrehungszahl des ersten Blendenkörpers größer ist als die des zweiten bedeutet das, daß der eine Schlitz im ersten Blendenkörper durchstrahlt wird, wenn der Schlitz bzw. der Blendenkörper der Strahlenquelle zugewandt ist, während ein benachbarter Schlitz durchstrahlt wird, während er bzw der Blendenkörper von der Strahlenquelle abgewandt ist. Der dabei erzeugte Röntgenstrahl ist im ersten Fall breiter als im zweiten Fall, wobei die Unterschiede um so ausgeprägter sind, je größer der Durchmesser des Blendenkörpers im Vergleich zu dessen Abstand von der Strahlenquelle ist.

Um auch in Fällen, in denen die genannte Voraussetzung nicht erfüllt ist, zu vermeiden, daß der Röntgenstrahl sich abwechselnd verbreitert und verengt, ist in weiterer Ausgestaltung der Erfindung vorgesehen, daß in dem zweiten Blendenkörper n Öffnungen vorgesehen sind, wobei n die Zahl der Schlitze in dem ersten Blendenkörper ist, daß die Öffnungen auf dem Umfang um einen Winkel von 180°/n gegeneinander versetzt sind, und daß ihre axiale Lage der axialen Lage jeweils eines Schlitzes entspricht, so daß die Strahlung jeweils und durch einen der Schlitze durch die ihm zugeordnete Öffnung tritt.

Dabei wird davon ausgegangen, daß die Röntgenstrahlung jeweils dann abgeschaltet wird, wenn die Schlitze im ersten Blendenkörper der Strahlenquelle zugewandt sind.

Die Erfindung wird nachfolgend anhand von Zeichnungen näher erläutert. Es zeigen:

Fig. 1 ein erstes Ausführungsbeispiel der Erfindung,

Fig. 2 den Blendenkörper und das Blendenteil bei einer ersten Ausführungsform,

Fig. 3 den Blendenkörper und das Blendenteil bei einer zweiten Ausführungsform,

Fig. 4 eine bevorzugte Ausführungsform im Querschnitt,

Fig. 5 bzw. Fig. 6 eine Draufsicht auf den bei der Ausführungsform nach Fig. 4 vorgesehenen ersten bzw Zweiten Blendenkörper und

Fig. 7 bzw. Fig 8 eine Abwicklung des ersten bzw. des zweiten Blendenkörpers

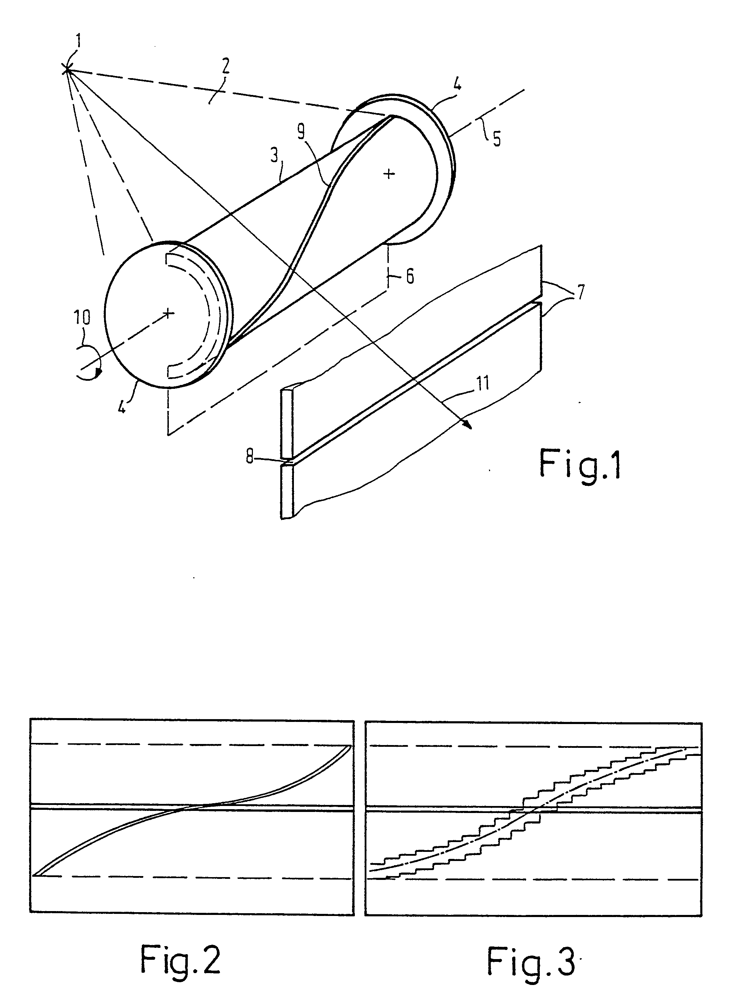

In Fig. 1 ist mit 1 der Fokus einer im übrigen nicht näher dargestellten Röntgenröhre bezeichnet, von dem ein durch gestrichelte Linien angedeutetes Röntgenstrahlbündel 2 ausgeht. Das Röntgenstrahlbündel 2 trifft auf einen Blendenkörper 3, der durch einen Hohlzylinder mit halbkreisförmigem Querschnitt gebildet wird. Der Blendenkörper ist an seinen Stirnflächen in kreisförmigen Scheiben 4 gehalten. Die Scheiben 4 sind durch einen nicht näher dargestellten Motor um eine Drehachse 5 drehbar, auf der die Krümmungsmittelpunkte der Innenfläche des Hohlzylinders 3 liegen. Wie durch die gestrichelte Linie 6 angedeutet, sind die Scheiben 4 in axialer Richtung unbeweglich. Dies kann beispielsweise dadurch erreicht werden, daß die Scheiben 4 um die Achse 5 drehbar in je einem Lager gelagert sind, das in einem mit dem Röntgenstrahler verbundenen, nicht näher dargestellten Gehäuse angeordnet ist.

Der Blendenkörper 3 besteht aus einem solchen Material und ist so dick, daß die Röntgenstrahlung dadurch praktisch vollständig absorbiert wird. Die Dicke und das Material des Blendenkörpers hängen von der Härte der Röntgenstrahlung ab. Bei einer Spannung an der Röntgenröhre von 120 kV absorbiert ein 1,5 mm dicker Blendenkörper aus Wolfram oder einer Wolframlegierung die Röntgenstrahlung praktisch vollständig. Die Dicke des Körpers 3 ist auf einen Wert $d_m$ begrenzt, der sich aus der Beziehung ergibt

$$d_m = w/\tan(\beta/2),$$

wobei ß der Ablenkwinkel des Röntgenstrahls und w die Breite des Röntgenstrahls ist. Für einen Ablenkwinkel von z.B. 23° und w = 0,5 mm ist $d_m$ = 2,45 mm.

Der Blendenkörper 3 ist mit einem schraubenlinienförmigen bzw. spiralförmigen Schlitz 9 versehen, der eine Ecke des Blendenkörpers (rechts oben) mit der gegenüberliegenden Ecke (links unten) verbindet. Die Breite des Schlitzes ist auf die Größe des auszublendenden Röntgenstrahls abgestimmt.

Jenseits des Blendenkörpers (vom Brennfleck 1 aus gesehen) befindet sich eine feststehende Blende, die einen geradlinigen Schlitz aufweist. Die Strahlenquelle 1, der Blendenkörper 3 und die Blende 7 sind vorzugsweise so relativ zueinander angeordnet, daß die Drehachse 5 des Blendenkörpers in der durch die Mittellinie des Schlitzes 8 und den Fokus 1 definierten Ebene liegt und parallel zu der durch die Blende 7 bestimmten Ebene verläuft. Der Fokus 1 soll möglichst in der zur Drehachse 5 senkrechten Symmetrieebene des Blendenkörpers 3 und der Blendenplatte 7 liegen.

Das Röntgenstrahlenbündel 2 durchdringt den Schlitz 9 in dem Blendenkörper an der Stelle, an der der Schlitz durch die durch die Drehachse 5 und den Fokus 1 bestimmte Ebene geschnitten wird sowie in einem gewissen Bereich um diese Schnittstelle herum. Die Blendenplatte 7 läßt jedoch nur den aus der Schnittstelle austretenden Röntgenstrahl hindurch und unterdrückt die beiderseits dieses Strahls aus dem Schlitz, d.h. oberhalb oder unterhalb der erwähnten Ebene, austretende Röntgenstrahlung weitgehend, so daß aus dem Schlitz 8 ein Röntgenstrahl 11 mit geringem Querschnitt (pencil beam) austritt.

Bei einer Drehung des Blendenkörpers um die Achse 5 entsprechend der durch den Pfeil 10 markierten Drehrichtung verschiebt sich der Schnittpunkt der erwähnten Ebene mit dem Schlitz 9 nach rechts, so daß der Röntgenstrahl 8 nach rechts auswandert, bis das rechte Ende des Schlitzes erreicht ist. Wird der Blendenkörper dann weiter gedreht, tritt der Röntgenstrahl zunächst durch das linke Ende des Schlitzes 9 hindurch und bewegt sich dann wieder nach rechts, so daß sich bei einer Drehung des Blendenkörpers mit konstanter Drehzahl eine periodische sägezahnförmige Bewegung des Röntgenstrahls ergibt.

Der Zeitraum zwischen dem Ende der Bewegung (rechts) und dem Wiederbeginn (links) hängt einerseits von dem Umfangswinkel ab, den der Schlitz 9 auf dem Blendenkörper beschreibt und andererseits von dem Bogen, den der Querschnitt des Körpers selbst beschreibt. Wenn der erwähnte Zeitraum kurz sein soll, darf der Bogen nur wenig größer sein als 180°, und der Winkel, den der Schlitz 9 auf dem Umfang des Blendenkörpers beschreibt, muß 180° betragen. Der Bogen kann nicht exakt zu 180° gemacht werden, weil in der Winkelstellung des Blendenkörpers, in der die Blendenkante in der durch die Drehachse 5 und den Fokus 1 definierten Winkelstellung liegt, Röntgenstrahlung an dem Blendenkörper vorbei durch den Schlitz 7 austreten würde. Infolgedessen muß der Bogen des kreisförmigen Querschnitts des Blendenkörpers etwas mehr als 180° betragen, so daß der Schlitz gegenüber dem Fokus 1 stets durch den Blendenkörper 7 abgeschirmt wird - bis auf die Stelle, an der der Röntgenstrahl 8 den Schlitz passiert.

Vorstehend wurde davon ausgegangen, daß der Schlitz 9 die Form einer Schraubenlinie hat, d.h. daß seine Steigung über die gesamte Länge des Zylinders konstant ist. In vielen Fällen ist es jedoch erwünscht, den Strahl in der Mitte schneller (oder langsamer) zu bewegen als am Rande. Eine solche Ausführungsform ist in Fig. 2 dargestellt, die die feststehende Blende 7 und den Blendenkorper 3 von der Seite der Blende 7 her zeigt. Man erkennt, daß die Steigung des Schlitzes (in Achsrichtung gesehen) in der Mitte größer ist als am Rande. Infolgedessen bewegt sich bei konstanter Drehzahl des Blendenkörpers der Röntgenstrahl in der Mitte schneller als am Rande.

Fig. 3 zeigt eine Ausführungsform, bei der der Schlitz 9 durch zwei treppenförmige Kurven 9a und 9b gebildet wird, wobei für alle Stufen der Treppe das Verhältnis von Breite zu Höhe konstant ist und im Verhältnis der Länge des Blendenkörpers zu seinem Umfang entspricht. Bei dieser Ausführungsform bewegt sich der ausgeblendete Röntgenstrahl nicht kontinuierlich, sondern sprungweise. Dies ist in verschiedenen Anwendungsfällen von Vorteil, beispielsweise bei solchen, bei denen die intensität des Primärstrahls (jenseits des Untersuchungsbereichs) mit einer Vielzahl nebeneinander angeordneter Detektoren gemessen wird.

Wenn der Blendenkörper einstückig ausgebildet ist, muß der Winkel, den der Querschnittsbogen des Blendenkörpers beschreibt, schon aus mechanischen Gründen stets größer sein als der Umfangswinkel, den der Schlitz beschreibt. Dadurch wird die Zeit vergrößert, die zwischen dem Verschwinden des Röntgenstrahls (am rechten Ende) und seinem Wiederauftauchen (am linken Rande) verstreicht. Außerdem ist die Herstellung eines Schlitzes in einem einstückig ausgebildeten Blendenkörper relativ kompliziert, insbesondere bei einem treppenförmigen Verlauf gemäß Fig. 3.

Diese Nachteile lassen sich dadurch vermeiden, daß der Blendenkörper aus zwei aneinander angepaßten Teilen hergestellt wird, die durch den Schlitz getrennt werden und die an ihren Stirnseiten fest mit den Scheiben 4 verbunden und vorzugsweise in dort vorgesehene kreisbogenförmige Nuten eingesetzt sind. Der gewünschte Verlauf des Schlitzes 1 läßt sich dann verhältnismäßig einfach durch entsprechende Bearbeitung der einander zugewandten Flächen der beiden den Blendenkörper bildenden Teile erreichen

Weil die Zentralprojektion (vom Brennfleck 1 aus) des Schlitzes 9 auf die Blendenplatte 7 den Schlitz 8 unter einem von 90° verschiedenen Win-

kel schneidet, hat der Querschnitt des Röntgenstrahls 11 bei gleicher Breite der Schlitze 8 und 9 in Richtung des Schlitzes größere Abmessungen als senkrecht dazu. Dieser Unterschied ist um so ausgeprägter, je kleiner der Durchmesser und je größer die Länge des Blendenkörpers 3 ist.

Eine deutlichere seitliche Begrenzung könnte dadurch erhalten werden, daß die Achse 5 aus der durch den Fokus 1 und den Schlitz 8 definierten Ebene gekippt würde, und zwar so, daß die rechte Seite des Blendenkörpers angehoben und die linke abgesenkt wird. Der Winkel zwischen der Zentralprojektion des Schlitzes 9 und dem Schlitz 8 würde dann nämlich größer, so daß der Röntgenstrahl 11 seitlich etwas schärfer begrenzt wäre. Allerdings müßte dann der Bogen des Blendenkörpers 3 noch weiter über 180° hinaus verlängert werden, um zu vermeiden, daß in einer Stellung des Blendenkörpers Röngtenstrahlung an diesem vorbei den Schlitz erreichen kann.

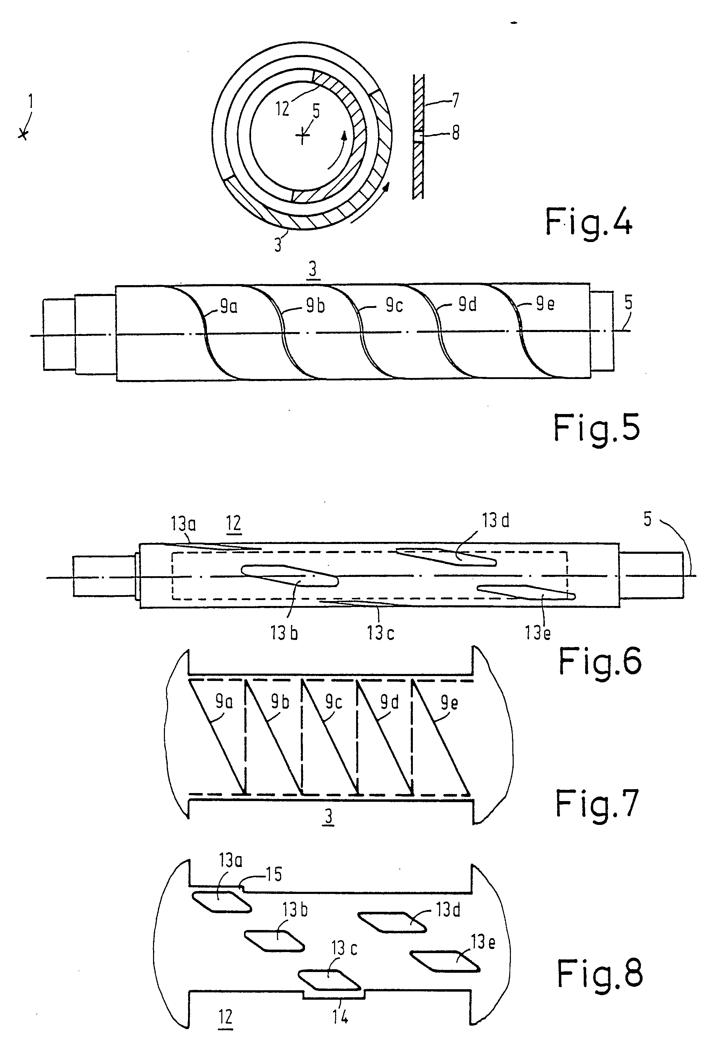

In Fig. 4 ist eine bevorzugte Ausführungsform dargestellt, die bei gleicher Länge und gleichem Durchmesser des Blendenkörpers eine wesentlich bessere seitliche Begrenzung des ausgeblendeten Röntgenstrahls 11 ergibt. Anstelle nur eines Blendenkörpers sind dabei zwischen der Strahlenquelle 1 und dem Blendenteil 7 ein erster 3 und ein zweiter 12 Blendenkörper koaxial und einander umschließend angeordnet. Die beiden Blendenkörper haben wiederum - jedenfalls über einen Teil ihrer Länge, der z.B 50 mm betragen kann - einen halbkreisförmigen Querschnitt. Ebenso wie bei der Anordnung nach Fig 1 ist zwischen der Strahlenquelle 1 und der Blendenanordnung eine nicht dargestellte Blende vorgesehen, die verhindert, daß Strahlung an der Blendenanordnung vorbei austreten kann.

Der erste Blendenkörper 3, der den zweiten Blendenkörper 12 umschließt, ist in den Fig. 5 sowie - in einer Abwicklungsdarstellung - in Fig. 7 dargestellt. Dieser erste Blendenkörper ist mit fünf Schlitzen 9a...9e versehen, die beispielsweise jeweils 0,4 mm breit sind. Es kann auch eine andere Zahl von Schlitzen vorgesehen sein, jedoch muß diese Zahl ungerade sein. Wie Fig. 7 deutlich zeigt, haben die Schlitze 9a...9e jeweils die gleiche, konstante Steigung. In axialer Richtung erstrecken sich die Schlitze jeweils über ein Fünftel der Länge des Bereichs mit halbkreisförmigem Querschnitt. Dabei gibt es eine gewisse Überlappung der in axialer Richtung von den Schlitzen eingenommenen Bereiche, d.h. der eine Schlitz beginnt bereits, wenn der benachbarte Schlitz noch nicht ganz geendet hat. Dadurch wird verhindert, daß innerhalb des konstruktiv vorgegebenen Ablenkwinkels des Röntgenstrahls eine Stelle nicht durchstrahlt wird. Der Querschnitt durch den ersten Zylinder 3 beschreibt einen Kreisbogen von genau 180°. Damit die

Schlitze den Blendenkörper nicht zerschneiden, erstrecken sie sich - wie Fig. 7 deutlich zeigt - über einen Umfangswinkel, der etwas kleiner ist als 180°, z.B. 170°.

Wie die Fig. 6 und 8 zeigen, die den inneren zweiten Blendenkörper 12 in einer Draufsicht und in einer Abwicklung darstellen, enthält der zweite Blendenkörper trapezförmige Öffnungen 13a...13e in einer Zahl, die der Zahl der Schlitze im ersten Blendenkörper entspricht; im Beispiel sind das also fünf Öffnungen Jede Öffnung erstreckt sich in axialer Richtung über einen Bereich, dessen Zentralprojektion auf den ersten Blendenkörper 3 mindestens so lang ist, wie jeweils ein Schlitz im ersten Blendenkörper (in axialer Richtung). Jede der Öffnungen 13a...13e erstreckt sich über einen Umfangswinkel, der breiter ist als ein Fünftel des Umfangswinkels, den die Schlitze 9a...9e im ersten Blendenkörper beschreiben, beispielsweise über 36°. Die Öffnungen sind auf dem Umfang um 180°/5, d.h. um 36°, gegeneinander versetzt. Die dem Schlitz 9a zugeordnete Öffnung 13a erstreckt sich über einen Winkel von 0-36°. Die dem zweiten Schlitz 9b zugeordnete Öffnung 13b erstreckt sich im Winkelbereich von 72 bis 108° und die dem mittleren Schlitz 9c zugeordnet Öffnung 13c über einen Winkelbereich von 144 bis 180°. Die Öffnung 13d nimmt den Winkelbereich von 36 bis 72° ein und die Öffnung 13e den Bereich von 108 bis 144°. Stege 14 und 15 im Bereich der außen liegenden Öffnungen 13a und 13c schließen die Öffnungen nach außen hin ab.

Die Außendurchmesser der Blendenkörper 3 bzw. 12 können beispielsweise 12,5 mm und 8,4 mm betragen und ihre mit Schlitzen bzw. Öffnungen durchsetzte axiale Länge ca. 50 mm. Dabei ist anzumerken, daß die Fig. 4 bzw. 5, 6 bzw. 7, 8 diese Blendenkörper jeweils mit unterschiedlichen Maßstäben darstellen.

Im Betrieb rotieren die Blendenkörper mit gleichem Umlaufsinn, wobei die Winkelgeschwindigkeit des ersten Blendenkörpers fünfmal so groß ist wie die des zweiten Blendenkörpers. Dies kann dadurch erreicht werden, daß an den Stirnflächen des Blendenkörpers nicht näher dargestellte Verzahnungen vorgesehen sind, die mit einem gemeinsamen Antriebsmotor gekoppelt sind, wobei das Übersetzungsverhältnis für die beiden Blendenkörper entsprechend gewählt ist. Stattdessen kann aber auch für jeden Blenden körper ein gesonderter Schrittmotor vorgesehen sein, wobei der Motor für den ersten Blendenkörper bei gleicher Schrittweite die fünffache Zahl von Antriebsimpulsen erhält oder - bei fünfmal so großer Schrittweite - die gleiche Impulszahl.

Es sei angenommen, daß in einem bestimmten Zeitpunkt des Betriebes die Schlitze 9a...9e dem Blendenteil 7 zugewandt sind und daß der Brennfleck sich in einer zur Zeichenebene der Fig. 6 senkrechten, die Rotationsachse 5 enthaltenden Ebene befindet. In diesem Fall kann ein Röntgenstrahl durch die Öffnung 13b, den Schlitz 9b und den Schlitz 8 im Blendenteil 7 hindurchtreten. Alle anderen Schlitze (genauer: die Teile dieser Schlitze, die in der durch den Strahler 1 und die Achse 5 definierten Ebene liegen) werden durch den Blendenkörper 12 abgeschirmt. Durch die Drehung verschiebt sich der Schnittpunkt des Schlitzes 9b mit der erwähnten Ebene nach rechts, d.h. der Röntgenstrahl wandert nach rechts aus, wobei sich gleichzeitig die Öffnung 13b nach oben bewegt, bis das Ende des Schlitzes 9b erreicht ist. Der Röntgenstrahl tritt dann gerade noch aus dem unteren Bereich der Öffnung 13b aus.

Nachdem auf diese Weise das Ende des Schlitzes 9b erreicht ist, wird bei weiterer Drehung die Strahlung durch den Schlitz 9b unterbrochen und die (in bezug auf die Drehrichtung) vordere Kante des ersten Blendenkörpers 3 schneidet die erwähnte Ebene. Danach würde ein anderer Schlitz von Röntgenstrahlung durchsetzt, nämlich derjenige, dessen zugeordnete Öffnung in Umfangsrichtung der zuletzt durchstrahlten Öffnung folgt; im Ausführungs beispiel wäre das der Schlitz 9e und die Öffnung 13e. Dabei wäre jedoch der Schlitz 9e der Strahlenquelle 1 zugewandt, d.h. er würde sich zwischen dez Strahlenquelle und der Achse 5 befinden, während der Schlitz 9b sich zuvor jenseits der Achse 5 befand. Bei gleicher Schlitzbreite würde des zur Folge haben, daß der Querschnitt des Röntgenstrahls verbreitert würde. Diese Änderung der Breite des ausgeblendeten Röntgenstrahls kann selbst dann noch störend sein, wenn bei einem Außendurchmesser des Blendenkörpers 3 von 12,5 mm der Abstand zwischen der Strahlenquelle 1 und der Achse 5 z.B. 110 mm beträgt.

Um die geschilderte Verbreiterung des Röntgenstrahls zu vermeiden, wird die Röntgenstrahlung ausgeschaltet, so lange sich die vordere Kante des Blendenkörpers unterhalb der durch 1 und 5 definierten Ebene befindet. Das Ausschalten kann durch einen nicht näher dargestellten Winkelgeber gesteuert werden, der mit dem Blendenkörper 5 gekoppelt ist.

Wenn die vordere Kante des Blendenkörpers 3 die erwähnte Ebene nach oben durchstößt, befindet sich die Oberkante der Öffnung 13c ebenfalls in dieser Ebene. Diese Öffnung gibt danach den Schlitz 9c frei, und der durch diesen Schlitz austretende Röntgenstrahl bewegt sich wiederum nach rechts. Wegen der geschilderten Überlappung der Schlitze 9a...9e in axialer Richtung tritt der Röntgenstrahl - bezogen auf die Ebene - an der gleichen Stelle aus, an der er bei Erreichen des Endes des Schlitzes 9b austrat bzw. noch etwas links davon. Dies könnte dazu führen, daß die Dosis des Röntgenstrahls in dem Überlappungsbereich größer ist als außerhalb, weil sich hier die Röntgenstrahlung am Ende des Schlitzes 9b bzw. am Anfang 9c addiert. Dieser Effekt läßt sich aber kompensieren, wenn in dieser durch den erwähnten Winkelgeber registrierten Stellung des Röntgenstrahls die Meßwerte der nicht näher dargestellten Detektoranordnung, die die durch ein nicht näher dargestelltes Objekt transmittierte bzw. davon gestreute Röntgenstrahlung erfaßt, mit einem geeigneten Gewichtungsfaktor multipliziert werden. Anschließend bewegt sich der Röntgenstrahl in dem durch den Schlitz 9c festgelegten Bereich von links nach rechts.

Danach wird die Röntgenstrahlung wieder ausgeschaltet, wonach die Öffnung 13d und der Schlitz 9d wirksam werden. Wenn sich die Öffnung 13c zuvor jedoch jenseits (in bezug auf die Strahlenquelle 1) der Achse 5 befand, befindet sich nun die Öffnung 13d zwischen der Achse 5 und der Strahlenquelle 1. Die Projektion der Öffnung 13d auf den Blendenkörper 3 wird dadurch zwar vergrößert, doch stört dies weiter nicht, weil der Querschnitt des ausgeblendeten Röntgenstrahls ausschließlich durch die Abmessungen eines der Schlitze 9a...9e und des Schlitzes 8 festgelegt sind. Die Öffnungen 13a.. 13e haben lediglich die Funktion, die Strahlung durch einen der Schlitze freizugeben.

Nachdem der Röntgenstrahl dann auch den durch den Schlitz 9d festgelegten Bereich durchfahren hat, wird nach Abschaltung der Röntgenstrahlung - für eine halbe Umdrehung der Schlitz 9 wirksam; danach der Schlitz 9a usw.. Auf diese Weise durchfährt ein von der Strahlenquelle emittierter Röntgenstrahl innerhalb von fünf aufeinanderfolgenden Umdrehungen einmal den mit Schlitzen versehenen Bereich im Blendenkörper 3. Die Öffnungen im Blendenkörper 12 können auch in anderer Weise auf dessen Umfang verteilt sein, wenn sie nur um 180°/n auf dem Umfang gegenüber benachbarten Öffnungen versetzt sind und den Abmessungen eines Schlitzes in axialer Richtung angepaßt sind. Allerdings werden dann räumlich aufeinanderfolgende Bereiche vom Röntgenstrahl nicht zeitlich nacheinander durchfahren, sondern dieser springt, beispielsweise vom Streifen 9c zum Streifen 9e oder in ähnlicher Weise.

Je kleiner das Verhältnis des Durchmessers des ersten Blendenkörpers 3 zum Abstand zwischen Strahlenquelle 1 und Rotationsachse 5 ist, desto weniger macht sich die weiter oben geschilderte abwechselnde Verbreiterung und Verengung des Röntgenstrahls bemerkbar. In Fällen, in denen dieses "Atmen' des Röntgenstrahles tolerierbar ist, kann darauf verzichtet werden, die Strahlenquelle 1 bei jeder zweiten halben Umdrehung des Blendenkörpers 3 abzuschalten. In diesen Fällen könnte der in den Fig. 6 und 8 dargestellte zweite Blendenkörper ebenfalls verwendet werden, wobei der Röntgenstrahl allerdings vom Schlitz 9a zum Schlitz 9d, von diesem zum Schlitz 9b usw springen würde. Jedoch kann dann der zweite Blendenkörper 12 auch wesentlich einfacher gestaltet sein. Es würde dann genügen, wenn er mit einem einzigen spiralförmigen breiten Schlitz versehen würde, der in der Darstellung der Fig. 8 von links oben nach rechts unten verläuft. Die Breite des Schlitzes muß dabei ausreichend sein, um das wandern des Röntgenstrahls innerhalb eines der Schlitze 9a...9e zu gestatten, aber nicht so groß, daß gleichzeitig zwei Schlitze in der durch 1 und 5 definierten Ebene mit Röntgenstrahlung beaufschlagt werden können. In diesem Fall kann die erste Blendenöffnung auch mit einer geraden Anzahl von Schlitzen versehen sein.

Es ist auch möglich, die Durchmesser der beiden Blendenkörper so zu wählen, daß der mit den Schlitzen versehene erste Blendenkörper im Innern des zweiten Blendenkörpers liegt. Das zuvor beschriebene "Atmen" des ausgeblendeten Röntgenstrahls bei ständig eingeschalteter Röntgenstrahlung ist dann weniger ausgeprägt; wenn die Röntgen strahlung jedoch bei jeder zweiten halben Umdrehung ausgeschaltet wird, ist es im allgemeinen zweckmäßiger, die in Fig 4 dargestellte umgekehrte Anordnung (erster Blenden körper außen) zu wählen, weil dann der aus den Schlitzen austretende Röntgenstrahl besser begrenzt ist.

## Ansprüche

1. Anordnung zur Erzeugung eines Röntgen- oder Gammastrahls (11) mit geringem Querschnitt und veränderlicher Richtung, mit einer ein Strahlenbündel liefernden Röntgen-oder Gammastrahlenquelle (1) und einer Blendenanordnung, die aus dem Strahlenbündel den Röntgenstrahl ausblendet, und die ein feststehendes Blendenteil (7) mit einem geradlinigen Schlitz (8) und einen um eine Drehachse (5) rotierenden zylinderförmigen ersten Blendenkörper (3) mit einem auf seinem Mantel spiralförmig umlaufenden Schlitz (9) umfaßt, dadurch gekennzeichnet, daß der Blendenkörper (3) zumindest über einen Teil seiner Länge einen wenigstens näherungsweise halbkreisförmigen Querschnitt hat.

2. Anordnung nach Anspruch 1, dadurch gekennzeichnet, daß die Drehachse (5) des Blendenkörpers (3) in der durch die Strahlenquelle (1) und den geradlinigen Schlitz (8) definierten Ebene liegt.

3. Anordnung nach einem der vorhergehenden Ansprüche, dadurch gekennzeichnet, daß der spiralförmige Schlitz (9) eine über die Länge des Blendenkörpers (3) variable Steigung hat (Fig. 2).

4. Anordnung nach einem der vorhergehenden Ansprüche, dadurch gekennzeichnet, daß der Schlitz einen stufenförmigen Verlauf hat (Fig. 3).

5. Anordnung nach einem der vorhergehenden Ansprüche, dadurch gekennzeichnet, daß in dem ersten Blendenkörper in axialer Richtung mehrere spiralförmig umlaufende Schlitze (9a...9e) aufeinander folgende

6. Anordnung nach Anspruch 5, dadurch gekennzeichnet, daß ein zweiter mit wenigstens einer Öffnung versehener Blendenkörper (12) mit halbkreisförmigem Querschnitt über wenigstens einen Teil seiner Länge vorgesehen ist, daß die beiden Blendenkörper (3, 12) koaxial und einander umschließend angeordnet sind, daß der erste Blendenkörper (3) entsprechend der Zahl der darin vorgesehenen Schlitze schneller rotiert als der zweite Blendenkörper (12), und daß die Anordnung und die Form der Öffnung(en) auf dem Umfang des zweiten Blendenkörpers derart ist, daß jeweils nur durch einen der Schlitze (z.B. 9b) ein nutzbarer Strahl austreten kann.

7. Anordnung nach Anspruch 6, dadurch gekennzeichnet, daß der erste (3) den zweiten Blendenkörper (12) umschließt.

8. Anordnung nach einem der vorhergehenden Ansprüche, dadurch gekennzeichnet, daß der erste Blendenkörper (3) zwischen der Strahlenquelle (1) und dem Blendenteil (7) angeordnet ist.

9. Anordnung nach einem der Ansprüche 7 oder 5, dadurch gekennzeichnet, daß in dem zweiten Blendenkörper n Öffnungen (13a...13e) vorgesehen sind, wobei n die Zahl der Schlitze in dem ersten Blendenkörper ist, daß die Öffnungen auf dem Umfang um einen Winkel von 180°/n gegeneinander versetzt sind, und daß ihre axiale Lage der axialen Lage jeweils eines Schlitzes (9a...9e) entspricht, so daß die Strahlung jeweils durch einen der Schlitze (z.B. 8b) und durch die ihm zugeordnete Öffnung (13b) tritt.

10. Anordnung nach Anspruch 5, dadurch gekennzeichnet, daß in dem zweiten Blendenkörper (12) als einzige Öffnung ein spiralförmiger Schlitz vorgesehen ist, der wesentlich breiter ist als die Schlitze in dem ersten Blendenkörper und der sich über einen Umfangswinkel von zumindest annähernd 180° erstreckt.

Fig.1

Fig.2

Fig.3

**Fig.4**

**Fig.5**

**Fig.6**

**Fig.7**

**Fig.8**